# EUROPÄISCHE PATENTSCHRIFT

(11) **EP 1 827 398 B1**
(45) Veröffentlichungstag und Bekanntmachung des Hinweises auf die Patenterteilung: **13.02.2013**
(21) Anmeldenummer: 05817999.5
(22) Anmeldetag: 08.12.2005
(51) Int. Cl.: A61K 9/70

(54) **TRANSDERMALES THERAPEUTISCHES SYSTEM MIT AKTIVIERBARER ÜBERSÄTTIGUNG UND KONTROLLIERTER PERMEATIONSFÖRDERUNG**
TRANSDERMAL, THERAPEUTIC SYSTEM WITH ACTIVATABLE OVERSATURATION AND CONTROLLED PERMEATION PROMOTION
SYSTEME THERAPEUTIQUE TRANSDERMIQUE A SURSATURATION ACTIVABLE ET PENETRATION CONTROLEE

(30) Priorität: 24.12.2004 DE 102004062614
(43) Veröffentlichungstag der Anmeldung: 05.09.2007
(73) Patentinhaber: LTS LOHMANN Therapie-Systeme AG, 56626 Andernach (DE)
(72) Erfinder: KOCH, Andreas, 56581 Melsbach (DE)
(74) Vertreter: Schweitzer, Klaus
(86) Internationale Anmeldenummer: PCT/EP2005/013133
(87) Internationale Veröffentlichungsnummer: WO 2006/072329

(56) Entgegenhaltungen:
- EP-A- 0 273 004
- EP-A- 0 384 266
- EP-A- 0 857 488
- US-A- 4 698 062
- US-A- 5 128 137

## Beschreibung

Die vorliegende Erfindung betrifft ein im Wesentlichen aus zwei Kompartimenten zusammengesetztes transdermales therapeutisches System (TTS) mit aktivierbarer Übersättigung und kontrollierter Permeationsförderung. Insbesondere betrifft die Erfindung ein TTS, bei dem eine übersättigte Wirkstofflösung in der wirkstoffhaltigen Polymermatrix erst bei Applikation des Systems auf der Haut erzeugt wird, hervorgerufen durch die kontrollierte Zufuhr einer oder mehrerer die Permeation des pharmazeutischen Wirkstoffs fördernder Substanzen (Permeationsenhancer). Die vorliegende Erfindung betrifft ferner die beiden Kompartimente, aus denen das erfindungsgemäße transdermale therapeutische System zusammengefügt wird, sowie die Herstellung dieses transdermalen therapeutischen Systems aus den zwei Kompartimenten.

Die Permeation von medizinischen Wirkstoffen durch die Haut in den Blutkreislauf, mit dem Ziel, physiologisch bzw. therapeutisch wirksame Plasmaspiegel oder systemisch pharmakodynamische Wirkungen zu erzielen, lässt sich in der Praxis oft nur unter Schwierigkeiten verwirklichen. Der Grund für die Schwierigkeiten ist die Haut selbst, die aufgrund ihres Aufbaus und ihrer Funktion eine wirksame Permeationsbarriere für transdermal zu applizierende Substanzen darstellt. Um dennoch Permeationsraten zu erzielen, mit denen physiologisch oder therapeutisch wirksame Plasmaspiegel erzeugt werden können, werden in der Praxis hauptsächlich 3 Möglichkeiten genutzt:
1. der Zusatz von die Permeation des Wirkstoffs fördernden Substanzen, so genannten Permeationsenhancern;
2. die Anwendung von elektrischem Strom (Iontophorese) und/oder Ultraschall (Phonophorese); sowie
3. die Verwendung von Freigabesystemen, in denen der Wirkstoff in einer Konzentration vorliegt, die über seine Löslichkeitsgrenze im entsprechenden Vehikel hinausgeht (übersättigte Systeme).

EP0384266 A2 beschreibt transdermale therapeutische Systeme enthaltend ein Wirkstoffkompartiment und ein Hilfsstoffkompartiment, welches Permeationsenhancer enthalten kann, wobei die zwei Kompartimente zum Zeitpunkt der Verabreichung so miteinander in Kontakt gebracht werden, dass der Hilfsstoff in das Wirkstoffreservoir gelangt.

Weitere Möglichkeiten, die transdermale Verabreichung eines Wirkstoffes zu verbessern, wie beispielsweise der Einsatz so genannter Prodrugs mit für die Hautpermeation günstigeren physikochemischen Eigenschaften (u.a. höhere Lipophilie), spielen bei der Entwicklung transdermaler Verabreichungssysteme nur eine untergeordnete Rolle.

Aber auch die in der Praxis hauptsächlich beschrittenen Wege zur Verbesserung der transdermalen Permeation von Wirkstoffen weisen beachtliche Nachteile auf.

Der wesentliche Nachteil bei einem Zusatz von Permeationsenhancern besteht darin, dass diese das Freigabesystem bei seiner Anwendung unkontrolliert und insbesondere in der Anfangsphase der Anwendung sehr schnell verlassen, da es sich bei diesen Enhancern in der Regel um leicht flüchtige organische Verbindungen handelt. Durch diese unkontrollierte Überdosierung des Permeationsenhancers (Enhancer-Dose-Dumping) kommt es häufig zu Hautreizungen. Zudem führt das Einarbeiten von Permeationsenhancern in die Matrix eines transdermalen therapeutischen Systems oft zu Stabilitätsproblemen, da der Wirkstoff mit den Permeationsenhancern in Wechselwirkung treten kann.

Der besondere Nachteil der Ionto- und Phonophorese liegt vor allem in deren Hautreizungspotential, da sowohl Strom als auch Ultraschall die Barrierefunktion der Haut stärker stören als chemische Permeationsenhancer.

Mit Wirkstoff übersättigte Freigabesysteme weisen den Nachteil auf, dass sie lediglich metastabil sind und die Rekristallisationsvorgänge in der wirkstoffhaltigen Matrix zu einer Verringerung der Bioverfügbarkeit des Wirkstoffs sowie zu einer Beeinträchtigung der Klebkraft von transdermalen therapeutischen Systemen führen können.

Zufriedenstellende Kompromisslösungen, bei denen die Übersättigung des Wirkstoffs in der Polymermatrix eines transdermalen therapeutischen Systems so hoch wie möglich, aber gleichzeitig so stabil wie erforderlich gehalten werden kann, sind nicht bekannt.

Der vorliegenden Erfindung lag daher die Aufgabe zugrunde, ein stabiles, übersättigtes transdermales therapeutisches System in Pflasterform bereitzustellen.

Die Aufgabe wird durch ein transdermales therapeutisches System gelöst, bei dem die Übersättigung einer polymeren Haftklebematrix mit Wirkstoff erst bei Applikation des Systems auf der Haut erzeugt wird und gleichzeitig Permeationsenhancer kontrolliert einwirken.

Es wurde überraschenderweise gefunden, dass sich eine kurzfristige Aktivierung eines transdermalen therapeutischen Systems zu einem übersättigten System mit maximaler thermodynamischer Aktivität sowie gleichzeitig stattfindender kontrollierter Wirkung zumindest eines Permeationsenhancers durch ein transdermales therapeutisches System gemäß Anspruch 1 erreichen lässt, welches erst bei seiner Anwendung aus zwei Kompartimenten zusammengesetzt wird, wobei der Wirkstoff in einem ersten Kompartiment, dem Verum-Kompartiment, in vollständig kristallisierter Form, d. h. in festem Aggregatzustand, in einer haftklebenden, polymeren Matrixschicht vorliegt und sich der/die Permeationsenhancer in einem zweiten Kompartiment (Enhancer-Kompartiment) befinden, bei dem es sich um ein Flüssig-Reservoirsystem handelt und das eine die Freisetzung des/der Permeationsenhancer(s) steuernde Membran aufweist.
Figur 1 ist eine schematische Darstellung von zwei Ausführungsformen des erfindungsgemäßen transdermalen therapeutischen Systems und der Kompartimente, aus denen es zusammengesetzt wird.
Figur 2 ist ein Diagramm zum Vergleich von Permeationsprofilen eines Verum-Kompartiments, das mit unterschiedlichen Enhancer-Kompartimenten, welche sich nur hinsichtlich der Steuermembran voneinander unterscheiden, kombiniert wurde.
Figur 3 umfasst zwei Diagramme zur Veranschaulichung von Versuchsergebnissen zur Auswahl von Membranen für die Kontrolle der Permeation eines Enhancers.
Figur 4 ist ein Diagramm zur Veranschaulichung der Wirkung von Absorptionsmitteln im Flüssigreservoir eines Enhancer-Kompartiments.

Im Folgenden wird die Erfindung unter Bezugnahme auf die Figuren und anhand von Beispielen näher erläutert, ohne dass dadurch die Erfindung in irgendeiner Weise eingeschränkt wird.

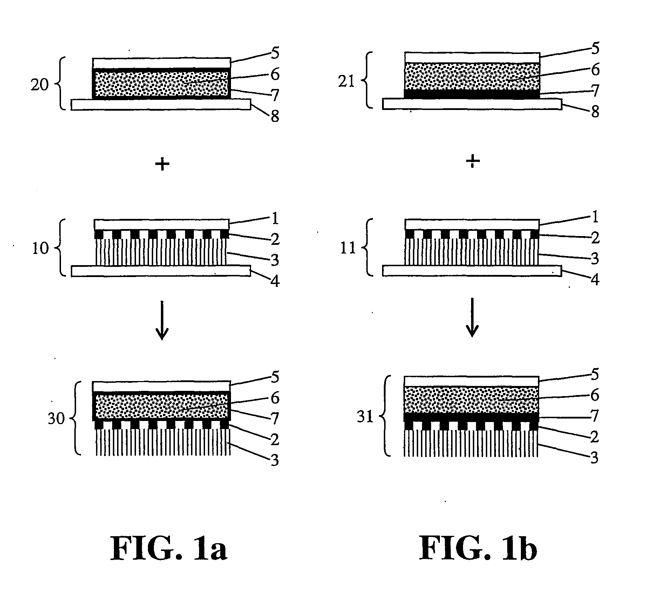

Das erfindungsgemäße transdermale therapeutische System (30, 31), wie in den Figuren 1a und 1b dargestellt, umfasst eine wirkstoffhaltige, haftklebende Polymermatrix (3), die auf ihrer der Haut abgewandten Seite mit einer perforierten oder nicht perforierten Rückschicht (2), welche eine hohe Permeabilität für den oder die Permeationsenhancer aufweist, versehen ist. Diese perforierte bzw. hoch permeable Rückschicht (2) wird auch als "innere Rückschicht" bezeichnet. Auf der der Polymermatrix (3) gegenüberliegenden Seite der inneren Rückschicht (2) befindet sich bei dem erfindungsgemäßen transdermalen therapeutischen System ein Flüssig-Reservoirsystem (6), welches eine Steuermembran (7) aufweist. Dabei kann die Hülle des Flüssig-Reservoirsystems (6) selbst, zumindest auf der dem Verum-Kompartiment zugewandten Seite, aus der Steuermembran (7) bestehen (Fig. 1a) oder die Steuermembran kann zusätzlich auf der der Polymermatrix (3) zugewandten Seite des Flüssig-Reservoirsystems (6) auf dessen Hülle aufgebracht sein (Fig. 1b), so dass die Steuermembran (7) zwischen dem Flüssigreservoir (6) und der inneren Rückschicht (2) angeordnet ist. Das transdermale therapeutische System (30, 31) umfasst zudem eine für Wirkstoff und Permeationsenhancer undurchlässige Rückschicht (5), die das TTS abgedeckt.

Das erfindungsgemäße TTS (30, 31) wird erst bei seiner Applikation aus zwei separat gefertigten Kompartimenten, einem Verum-Kompartiment (10, 11) und einem Enhancer-Kompartiment (20, 21), zusammengesetzt, nachdem die wiederablösbare, äußere Rückschicht (1) vom Verum-Kompartiment entfernt wurde.

Das Verum-Kompartiment (10, 11) umfasst eine haftklebende Polymermatrix (3), in welcher der Wirkstoff völlig durchkristallisiert, also im festen Aggregatzustand, vorliegt. Die haftklebende Polymermatrix (3) wird auf einer ihrer beiden Seiten von der inneren Rückschicht (2) abgedeckt, die perforiert ist oder eine hohe Permeabilität für den/die zu verwendenden Permeationsenhancer im Enhancer-Kompartiment aufweist. Die innere Rückschicht (2) wird ihrerseits auf ihrer der haftklebenden Polymermatrix (3) gegenüberliegenden Seite von der sogenannten äußeren Rückschicht (1) abgedeckt. Die äußere Rückschicht (1) ist wirkstoffundurchlässig und von der inneren Rückschicht (2) ablösbar ausgebildet. Die haftklebende Polymermatrix (3) wird auf ihrer der inneren Rückschicht (2) gegenüberliegenden Seite von einer wirkstoffundurchlässigen, ablösbaren Schutzschicht (4) abgedeckt.

Die haftklebende Matrixschicht (3) kann aus haftklebenden Polymeren auf Basis von Acrylsäure und/oder Methacrylsäure sowie deren Estern, Polyacrylaten, Isobutylen, Ethylen-Vinylacetat, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymere oder Heißschmelzklebern bestehen. Die Matrixschicht kann auch auf Basis von haftklebenden Silikonpolymeren oder Polysiloxanen hergestellt werden; besonders bevorzugt werden aminresistente Polydimethylsiloxane. Diese Aufzählung ist bei weitem nicht vollständig, lässt aber die breite Anwendungsfähigkeit des erfindungsgemäßen Prinzips erkennen.

Für die innere Rückschicht (2) können Folien verwendet werden, über die der/die mit dem Enhancer-Kompartiment zuzuführenden Permeationsenhancer in die wirkstoffhaltige Polymermatrix diffundieren kann/können, d. h. Folien, die für den/die Permeationsenhancer permeabel sind. Für die innere Rückschicht (2) können aber auch Folien verwendet werden, die für den/die Permeationsenhancer und möglicherweise auch für den Wirkstoff und/oder weitere Hilfsstoffe undurchlässig sind, sofern diese Folien mittels geeigneter Walzen- oder Stanzwerkzeuge perforiert wurden. Bevorzugt wird Polyesterfolie verwendet, die mit kleinen Löchern versehen wurde, welche vorzugsweise einen Durchmesser von 1,0 mm haben und einen ebenso großen Abstand zu den benachbarten Löchern aufweisen.

Als Material für die äußere Rückschicht (1) kommen solche Folien in Betracht, die von der inneren Rückschicht (2) ohne Probleme wieder ablösbar sind. Geeignet sind vor allem Polyester, welche sich durch besondere Festigkeit auszeichnen. Darüber hinaus können aber auch nahezu beliebige andere hautverträgliche Kunststoffe verwendet werden, wie z. B. Polyvinylchlorid, Ethylenvinylacetat, Vinylacetat, Polyethylen, Polypropylen, Cellulosederivate und viele andere mehr. Falls erforderlich kann die Ablösbarkeit der Folie durch eine geeignete Behandlung, z. B. Silikonisierung, zumindest ihrer der inneren Rückschicht (2) zugewandten Oberfläche bewirkt werden. Im Einzelfall kann die Folie mit einer zusätzlichen Auflage versehen werden, z. B. durch Bedampfung mit Metallen oder anderen diffusionssperrenden Zusatzstoffen wie Siliciumdioxid, Aluminiumoxid oder ähnlicher Stoffe, die dem Fachmann bekannt sind. Besonders bevorzugt werden Haftfolien auf Basis von Polyurethan (Opraflex^{®}), Polyisobutylen oder Polyacrylaten, wenn die innere Rückschicht eine Folie auf Basis von Polyethylenterephtalat (PET) ist.

Für die ablösbare Schutzschicht (4) können dieselben Materialien verwendet werden wie für die äußere Rückschicht (1), vorausgesetzt, dass sie durch geeignete Oberflächenbehandlung, wie z. B. Silikonisierung, ablösbar ist. Es können aber auch andere ablösbare Schutzschichten, wie Polytetrafluorethylen-behandeltes Papier, Cellophan, Polyvinylchlorid, oder ähnliche verwendet werden.

Bei dem Enhancer-Kompartiment (20, 21) handelt es sich um ein Flüssig-Reservoirsystem (6), auch Beutelsystem genannt, bei dem der oder die Permeationsenhancer in flüssiger Form, als Gel, Paste oder Lösung vorliegen.

Das Enhancer-Kompartiment (20, 21) weist eine Steuermembran (7) auf, mit der die Freisetzung des/der Permeationsenhancer(s) aus dem Flüssig-Reservoirsystem kontrolliert wird, sowie eine für den/die Permeationsenhancer und Wirkstoff undurchlässige Rückschicht (5) und eine ebensolche ablösbare Schutzfolie (8).

Die Wahl des/der Permeationsenhancer richtet sich nach ihrer Hautverträglichkeit und dem jeweiligen Wirkstoff. Dieser sollte in dem oder zumindest einem der Permeationsenhancer zumindest teilweise löslich sein, besser ist natürlich eine gute Löslichkeit. Als geeignete Enhancer-Komponenten können verwendet werden:
- niedere einwertige Alkohole wie beispielsweise Ethanol
- höhere einwertige Alkohole wie beispielsweise Oktanol
- mehrwertige Alkohole wie beispielsweise Butandiol
- monosubstituierte Ester von mehrwertigen Alkoholen wie beispielsweise Glycerinmonooleat oder Diethylglycolmonoethylether
- pharmazeutisch verträgliche Terpene oder Terpenalkohole wie beispielsweise Limonen bzw. Eucalyptol
- Ester mittlerer Carbonsäuren wie beispielsweise Diethylsebacat, Methyllaurat oder Lauryllactat
- Dimethylsulfoxid
- Ölsäure
- Dimethylisosorbid
- Derivate von Polyoxyethylenfettalkoholen wie beispielsweise Carbowax-350^{®}
- Derivate von Polyoxyethylenfettalkoholethern wie beispielsweise Brij 30^{®}
- Derivate von Polyoxyethylenfettsäureestern wie beispielsweise Tween 20^{®}
- Partialfettsäureester der Sorbitane wie beispielsweise Span 20^{®}
- pharmazeutisch verträgliche flüssige N₂-Verbindungen wie beispielsweise N-Methylpyrrolidon, Diethyltoluamid, Dimethylenpropylenharnstoff oder Diethanolamin
bzw. Mischungen dieser Komponenten miteinander.

Die Steuerung für die kontrollierte Freisetzung des/der Permeationsenhancer aus dem Enhancer-Kompartiment (20, 21) kann erreicht werden über die:
- Art (chemische Zusammensetzung und Porengröße) der eingesetzten Steuermembran und /oder
- Art (chemische Zusammensetzung und Schichtdicke) der eingesetzten Haftkleberschicht unter der Steuermembran, mit der das Flüssig-Resservoirsystem des Enhancer-Kompartiments versehen ist, um das Enhancer-Kompartiment auf dem Verum-Kompartiment zu befestigen, und/oder
- verzögerte Abgabe durch den Einsatz von Absorptionsmitteln im Flüssig-Reservoirsystem, beispielsweise durch Cyclodextrine oder Polyvinylpyrrolidone oder Cellulosederivate.

Als Steuermembran sind dünne Polymerfolien aus Polyethylen (z.B. Solupor^{®}), Polypropylen (z.B. Celgard^{®}), Polyurethan (z.B. Opraflex^{®}), Copolymeren aus Polyethylen und Polyvinylacetat (z.B. EVA^{®}) und Silikonen (z.B. Silastic^{®}) geeignet.

Dabei kann die Hülle des Flüssig-Reservoirsystems (6) selbst, zumindest auf der später dem Verum-Kompartiment zugewandten Seite, aus der Steuermembran (7) bestehen (Fig. 1a) oder die Steuermembran kann zusätzlich auf der der Polymermatrix (3) zugewandten Seite des Flüssig-Reservoirsystems (6) auf dessen Hülle aufgebracht sein (Fig. 1b), so dass die Steuermembran (7) zwischen dem Flüssigreservoir (6) und der inneren Rückschicht (2) angeordnet ist.

Als Haftkleber mit steuernden Eigenschaften sind vor allem solche auf Basis von Copolymeren aus Polyethylen und Polyvinylacetat mit Klebharzen als Zusätze geeignet. Über das Verhältnis Polyethylen zu Polyvinylacetat kann die Durchlässigkeit bzw. Permeabilität einer solchen Haftmatrix eingestellt werden. Geeignet sind auch Haftkleber auf Basis von Silikonen, da diese sehr gut diffusibel für die meisten Wirk- und Hilfsstoffe sind.

Das Flüssigreservoir-System des Enhancer-Kompartiments kann neben dem/den Permeationsenhancer(n) auch viskositätserhöhende Zusatzstoffe (Verdickungsmittel) enthalten, die keine Steuerfunktion haben. Geeignete Verdickungsmittel sind beispielsweise feindisperses Siliziumoxid wie z.B. Aerosil R 974^{®}, Polyacrylsäure wie z.B. Carbopol 934P^{®}, Mineralöle, Wollwachs oder hochmolekulare Polyethylenglykole wie z.B. Carbowax 1000^{®}.

Für die ablösbare Schutzfolie (8) kommen die gleichen Materialien in Betracht wie für die ablösbare Schutzschicht (4) oder die äußere Rückschicht (1) des Verum-Kompartiments, wobei sicherzustellen ist, dass das Material auch nicht mit nur einer der Komponenten im Flüssig-Reservoirsystem reagiert.

Die einzelnen Kompartimente des erfindungsgemäßen TTS sind bei Lagerbedingungen stabil und frei von unerwünschten Nebenprodukten, da keine Übersättigung vorliegt und somit das Problem der Metastabilität bzw. die Rekristallisationsneigung während der Lagerung gar nicht erst auftreten kann. Die Rekristallisationsvorgänge während der Anwendungsdauer des TTS haben keinen Einfluss auf die Bioverfügbarkeit des Wirkstoffs, sofern die Diffusionsgeschwindigkeit des Wirkstoffs kleiner ist als seine Freisetzungsgeschwindigkeit aus den Kristallen bzw. dessen Lösungsgeschwindigkeit.

Das erfindungsgemäße TTS wird erst unmittelbar bei dessen Anwendung hergestellt, indem das Enhancer-Kompartiment als Überpflaster auf das Verum-Kompartiment geklebt wird. Dazu wird vorzugsweise zunächst das Verum-Kompartiment nach Entfernen seiner Schutzschicht (4) auf der Haut befestigt und anschließend dessen äußere Rückschicht (1) abgezogen. Das Enhancer-Kompartiment wird dann nach Entfernen seiner Schutzfolie (8) auf das Verum-Kompartiment geklebt.

Direkt nach dem Aufbringen des Enhancer-Kompartiments auf das Verum-Kompartiment kann der Permeationsenhancer oder können die Permeationsenhancer kontrolliert in das Verum-Kompartiment diffundieren. Die ersten Wirkstoffkristalle werden unmittelbar nach Applikation des Enhancer-Kompartiments gelöst und es liegt sofort eine übersättigte Wirkstoff-Lösung mit höchster thermodynamischer Aktivität in der Matrix vor. Diese thermodynamische Aktivität bleibt auch sehr lange stabil, da das Lösemittel (= Permeationsenhancer) kontrolliert in das Wirkstoff-Donor-Kompartiment (Verum-Kompartiment) abgegeben wird.

Die vorliegende Erfindung kombiniert somit die Vorteile von Flüssig-Reservoirsystemen und matrixkontrollierten TTS-Systemen bei gleichzeitigem Ausschalten ihrer Nachteile (z. B. begrenzte Wirkstoff- und Enhancer-Beladungskapazitäten bei Matrix-Systemen, hohes Hautreizungspotential aufgrund schneller und großer Enhancer-Freisetzungen und Gefahr des Dose-Dumping bei Flüssig-Reservoirsystemen).

### Beispiel 1: Herstellung eines Verum-Kompartiments

In 80 g eines Lösemittelgemisches, bestehend aus 2 Teilen Spezialbenzin Typ 80/110 und 1 Teil Propylacetat, werden 54 g eines EVA-Copolymeren mit 40 Gew.-% Vinylacetat und einem Schmelzindex von 55 (EVATANE 40/55^{®}) eingetragen und unter Rühren sowie Wärmezufuhr bei 50°C gelöst. Nach ca. 30 min Rührzeit wurde eine viskose, farblos bis leicht trübe Lösung erhalten. In diese Lösung wurden anschließend 66 g des Klebharzes Foral^{®} 85 E eingetragen und bis zum vollständigen Auflösen (ca. 15 min) bei ebenfalls 50°C gerührt. Es resultierte eine 45,7%ige, niedrig viskose, gelbliche und leicht trübe Lösung (Kleberlösung A), die auch nach dem Erkalten noch als rührfähige Kleberlösung vorlag.

Für die Herstellung der selbstklebenden, wirkstoffhaltigen Matrix wurden 8,75 g Kleberlösung A vorgelegt, in die unter Rühren portionsweise 1,0 g Moxonidin-Base, ein lipophiler, schwer wasserlöslicher Arzneiwirkstoff, eingetragen wurde. Dieser Masseansatz wurde insgesamt 30 Minuten lang bei einer Rührgeschwindigkeit von 350 U/min homogenisiert. Anschließend erfolgte eine viertelstündige Entgasung bei 45 °C im Ultraschallbad, um überschüssige Luft aus der Masse zu entfernen.

Die wirkstoffhaltige Klebermasse wurde dann mit Hilfe einer Ausziehrakel in einer Naßschichtdicke von 300 µm auf einer silikonisierten Polyethylentherephtalat-Folie ausgestrichen. Danach wurden die Lösemittel durch halbstündiges Trocknen bei 50°C in einem Trockenschrank mit Abluftführung entfernt.

Anschließend wurde der lösemittelfreie, wirkstoffhaltige Klebefilm mit einer 15 µm dicken, wirk- und hilfsstoffundurchlässigen Polyesterfolie durch Aufkaschieren abgedeckt, wobei die Polyesterfolie zuvor mittels eines geeigneten Walzen- bzw. Stanzwerkzeugs dergestalt perforiert worden war, dass sowohl die Lochgröße als auch der Lochabstand in alle Richtungen 1,0 mm betrug.

Die perforierte Abdeckschicht (innere Rückschicht) wurde zwecks Lagerung des Verum-Kompartiments zusätzlich mit einer 15 µm dicken, wirk- und hilfsstoffundurchlässigen Folie aus Polyester (äußere Rückschicht), die zur reversiblen Befestigung auf der inneren Rückschicht auf einer ihrer Seiten mit einer Haftkleberschicht aus Polyisobutylen (Oppanol^{®} B10/B100) beschichtet war, kaschiert.

Der Wirkstoffanteil in der haftklebenden Matrix nach Fertigungsende betrug 20 Gew.-%; der Wirkstoff lag in dem Verum-Kompartiment völlig kristallisiert vor.

### Beispiel 2: Herstellung von Enhancer-Kompartimenten mit selbstklebender Steuermembran

Für die Herstellung des selbstklebenden Enhancer-Kompartiments (Flüssigreservoir-System) wurde zunächst die wirkstofffreie Kleberlösung A (Beispiel 1) mit Hilfe einer Ausziehrakel in einer Nassschichtdicke von 300 µm auf einer silikonisierten Polyethylentherephtalat-Folie ausgestrichen. Danach wurden die Lösemittel durch halbstündiges Trocknen bei 50°C in einem Trockenschrank mit Abluftführung entfernt. Der lösemittel- und wirkstofffreie Klebefilm wurde anschließend mit einer 35 µm dicken Polyurethanfolie (Opraflex^{®}, Fa. Lohmann, Deutschland) (erfindungsgemäße Ausführungsform EB1) bzw. mit einer 25 µm dicken Polypropylenfolie (Celgard X-20^{®}, Celanese Separation Products, USA) (erfindungsgemäße Ausführungsform EB2) durch Aufkaschieren abgedeckt. Bei diesen Folien handelt es sich um die späteren Steuermembranen. Auf diese Opraflex- bzw. Celgard-Folie wurde dann eine Polyesterfolie (Scotchpak No. 1220^{®}, Fa. 3M, Deutschland) aufgelegt, und mittels einer speziellen Siegelmaske, die über ein handelsübliches Bügeleisen aufgeheizt wurde, wurden Beutel gefertigt, die ein rundes Reservoir mit einem Durchmesser von 25 mm aufwiesen.

Über eine vorhandene Öffnung im Siegelrand der Beutel wurde die jeweilige Enhancermischung mittels einer Spritze in das Reservoir eingefüllt. Nach dem Füllen wurde die Einfüllöffnung mit Hilfe des Bügeleisens verschweißt, so dass ein komplett geschlossenes und lagerstabiles Flüssig-Reservoirsystem entstand.

### Beispiel 3: Permeationsprofile von TTS

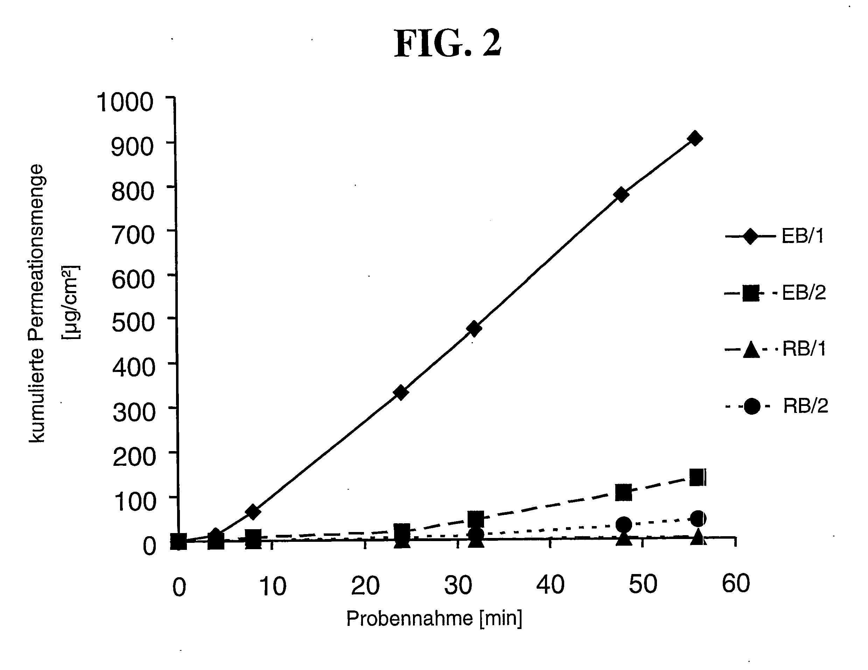

Um die Permeationseigenschaften erfindungsgemäßer TTS zu untersuchen und miteinander vergleichen zu können, wurden Permeationsmessungen am in vitro-Diffusionsmodell der Human-Vollhaut mit Hilfe modifizierter Franz-Diffusionszellen durchgeführt. Die Versuchsergebnisse sind in Figur 2 graphisch dargestellt.

Als Akzeptormedium diente isotonische Kochsalz-Lösung mit Zusatz von 0,1 % NaN₃ als Konservierungsmittel, thermostatisiert auf 32 °C.

Als Enhancermischung im Reservoir des Enhancer-Kompartiments wurden 300 mg einer Mischung von Ethanol : Ölsäure : N-Methylpyrrolidon im Verhältnis 2 : 1,5 : 1,5 (V/V/V) verwendet.

Für die Ausführungsform EB/1 eines erfindungsgemäßen TTS wurde das Enhancer-Kompartiment EB1 gemäß Beispiel 2 nach Entfernen der silikonisierten Polyethylentherephtalat-Folie als Überpflaster auf einem Verum-Kompartiment gemäß Beispiel 1, von dem zuvor die äußere Rückschicht abgezogen worden war, befestigt.

Als alternative Ausführungsform EB/2 eines erfindungsgemäßen TTS wurde an Stelle des Enhancer-Kompartiments EB1 das Enhancer-Kompartiment EB2 gemäß Beispiel 2 als Überpflasster verwendet.

Als Referenz RB/1 diente ein Verum-Kompartiment gemäß Beispiel 1, bei dem die auf die Matrix aufkaschierte 15 µm dicke, wirk- und hilfsstoffundurchlässige Polyesterfolie nicht perforiert worden war, zudem keine weitere, äußere Rückschicht aufwies und in den Versuchen auch ohne Enhancer-Überpflaster verwendet wurde.

Als weitere Referenz (RB/2) diente ein herkömmliches matrixkontrolliertes TTS mit Enhancer.

Figur 2 veranschaulicht die gegenüber den Referenzbeispielen RB/1 und RB/2 höhere Permeation des Wirkstoffs bei den erfindungsgemäßen Ausführungsformen EB/1 und EB/2.

### Beispiel 4: Einfluss der Steuerungsmembran auf die Permeation von Enhancern

Um den Einfluss der Steuerungsmembran auf die Permeation von Enhancern zu untersuchen, wurden Permeationsmessungen am in vitro-Diffusionsmodell Polyurethan-Membran mit Hilfe modifizierter Franz-Diffusionszellen durchgeführt, bei denen eine nichtklebede Polyurethanmembran (Opraflex®, Fa. Lohmann, DE) als Diffusionsmembran verwendet wurde. Als Akzeptormedium diente in allen Fällen isotonische Kochsalz-Lösung mit Zusatz von 0,1% NaN₃ als Konservierungsmittel, thermostatisiert auf 32 °C.

Als Enhancermischung wurden jeweils 300 µl Ethanol : Ölsäure : N-Methylpyrrolidon im Verhältnis 2 : 1,5 : 1,5 (v/v/v) auf die sich auf der Diffusionsmembran befindliche Membran aufgetragen.

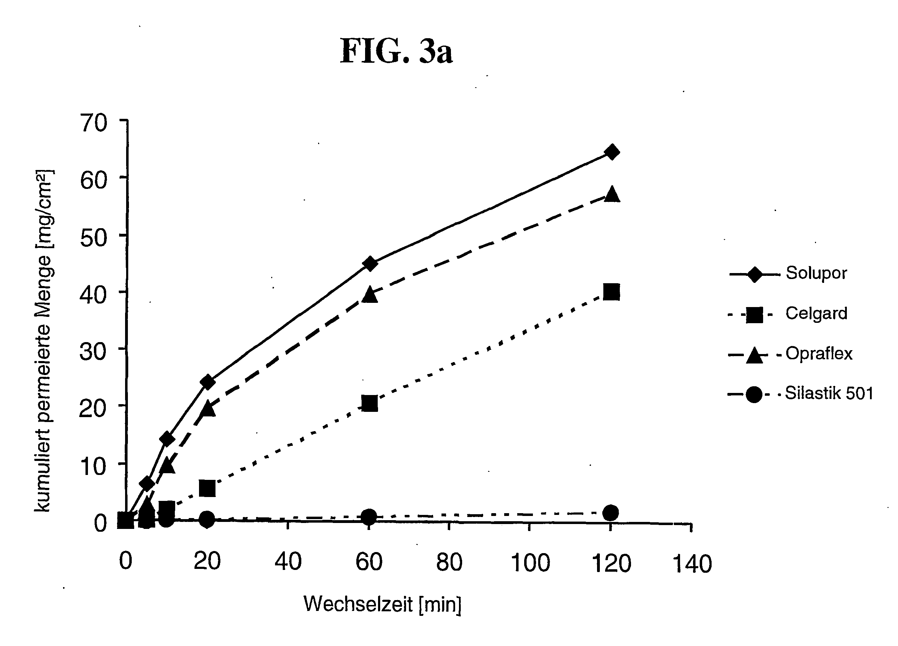

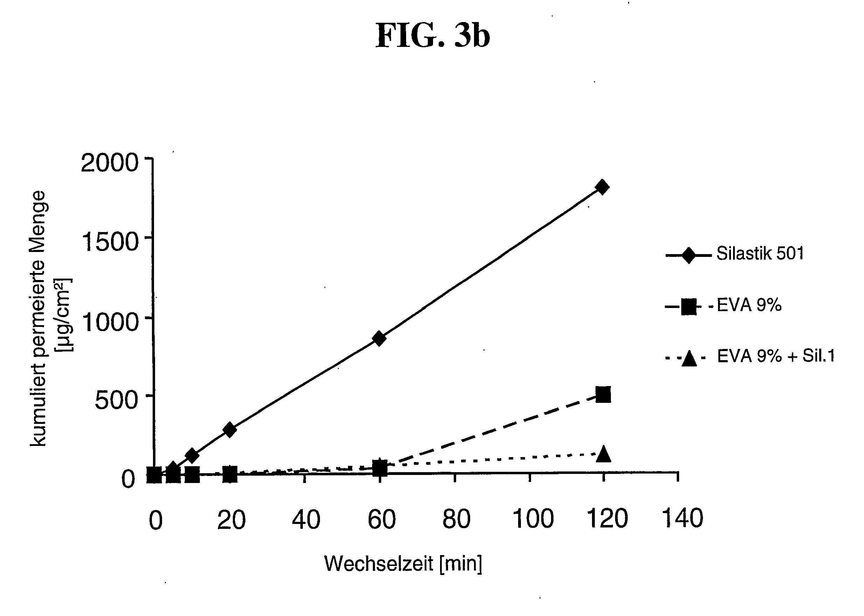

Die als Steuermembran verwendeten Membranen sind den Legenden der Diagramme in den Figuren 3a und 3b zu entnehmen. Alle Steuermembranen waren mit einer 300 µm dicken Haftkleberbeschichtung auf Basis von Copolymeren aus Polyethylen und Polyvinylacetat (Kleberlösung A gemäß Beispiel 1) ausgestattet, über die sie an der Diffusionsmembran befestigt waren. Die Diagramme in den Figuren 3a und 3b selbst veranschaulichen die Permeation von N-Methylpyrrolidon in Abhängigkeit der gewählten Steuermembran.

### Beispiel 5: Einfluss des Absorptionsmittels im Flüssigreservoir des Enhancer-Kompartiments auf die kontrollierte Enhancer-Permeation, gemessen am Enhancerbeispiel N-Methylpyrrolidon

Die Permeationsmessungen wurden am in vitro-Diffusionsmodell Polyurethan-Membran mit Hilfe modifizierter Franz-Diffusionszellen, bei denen eine nichtklebede Polyurethan-membran (Opraflex®, Fa. Lohmann, DE) als Diffusionsmembran verwendet wurde, durchgeführt. Als Akzeptormedium diente isotonische Kochsalz-Lösung mit 0,1% Zusatz von NaN₃ als Konservierungsmittel, thermostatisiert auf 32 °C.

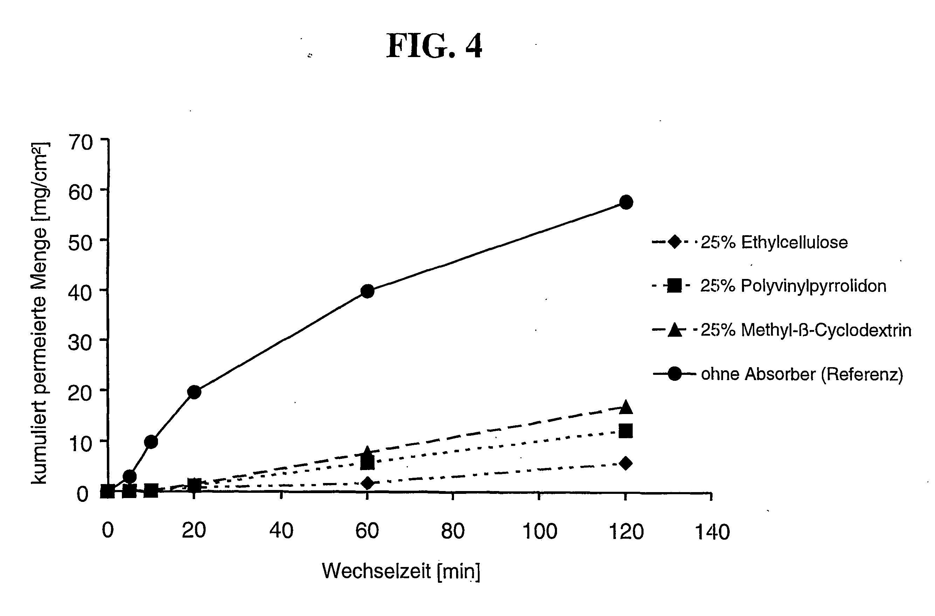

Als Enhancermischung wurden 800 mg Ethanol : Ölsäure : N-Methylpyrrolidon 2 : 1,5 : 1,5 (V/V/V), gemischt mit den aus der Legende zu Figur 4 ersichtlichen Absorptionsmitteln, direkt auf die Steuermembran (Celgard^{®} mit Haftkleberschicht) aufgetragen.

Das Diagramm in Figur 4 veranschaulicht den Einfluss verschiedener Absorptionsmittel auf die Permeation von N-Methylpyrrolidon.

## Patentansprüche

1. Transdermales therapeutisches System (TTS), umfassend ein erstes Kompartiment und ein zweites Kompartiment, wobei
- das erste Kompartiment eine haftklebende Polymermatrix umfaßt, die auf ihrer Haut abgewandten Seite mit einer inneren Rückschicht versehen ist und in welcher ein im festen Aggregatszustand vorliegender Wirkstoff enthalten ist, und deren eine Seite der Befestigung des Systems auf der Haut dient und mit einer wirkstoffundurchlässigen, ablösbaren Schutzschicht bedeckt ist, die sich auf der der inneren Rückschicht gegenüberliegenden Seite befindet;
- das zweite Kompartiment ein Flüssigkeits-Reservoirsystem umfaßt, das einen oder mehrere Permeationsenhancer in flüssiger Form, als Gel, Paste oder Lösung enthält, wobei der genannte Wirkstoff in dem Permeationsenhancer oder zumindest in einem der Permeationsenhancer mindestens teilweise löslich ist;
und wobei die beiden Kompartimente vor der Anwendung voneinander getrennt aufbewahrt werden und erst zur Anwendung auf der menschlichen Haut derart zusammengefügt werden, daß diejenige Seite des ersten, wirkstoffhaltigen Kompartiments, welche der mit der ablösbaren Schutzschicht bedeckten Seite gegenüberliegt, mit dem zweiten, Enhancer enthaltenden Kompartiment verbunden wird und die Diffusion des/der Enhancer in das wirkstoffhaltige Kompartiment über eine Steuermembran kontrolliert ermöglicht wird, so daß ein kontinuierlich übersättigtes System mit maximaler thermodynamischer Aktivität ausgebildet wird;
und wobei Verum-Kompartiment und Enhancer-Kompartiment nach ihrem Zusammenfügen durch eine "innere Rückschicht" voneinander getrennt sind, bei der es sich um eine Schicht mit hoher Permeabilität für den/die Permeationsenhancer handelt.

2. TTS nach Anspruch 1, **dadurch gekennzeichnet, dass** der/die Permeationsenhancer aus dem Enhancer-Kompartiment ausgewählt sind aus der Gruppe, die niedere einwertige, höhere einwertige und mehrwertige Alkohole, monosubstituierte Ester von mehrwertigen Alkoholen, Terpene, Terpenalkohole, Ester mittlerer Carbonsäuren, Polyoxyethylenfettalkohole, Polyoxyethylenfettalkoholether, Polyoxyethylen-fettsäureester, Partialfettsäureester der Sorbitane, Dimethylisosorbid, Dimethylsulfoxid, Ölsäure sowie pharmazeutisch verträgliche flüssige Stickstoffverbindungen wie N-Methylpyrrolidon, Diethyltoluamid, Dimethylenpropylenharnstoff, Diethanolamin umfaßt.

3. TTS nach Anspruch 2, **dadurch gekennzeichnet, dass** die Permeationsenhancer aus dem Enhancer-Kompartiment einzeln oder als Mischungen untereinander vorliegen.

4. TTS nach einem der Ansprüche 1 bis 3, **dadurch gekennzeichnet, dass** die Permeationsenhancer aus dem Enhancer-Kompartiment über eine Steuermembran kontrolliert in den Verum-Kompartiment abgegeben werden.

5. TTS nach Anspruch 4, **dadurch gekennzeichnet, dass** die Hülle des Flüssig-Reservoirsystems, zumindest auf der dem Verum-Kompartiment zugewandten Seite, aus der Steuermembran besteht, oder zusätzlich auf der dem Verum-Kompartiment zugewandten Seite des Flüssig-Reservoirsystems aufgebracht ist.

6. TTS nach Anspruch 4 oder 5, **dadurch gekennzeichnet, dass** die Steuermembran selbstklebend oder mit einer Haftkleberschicht zur Befestigung des Enhancer-Kompartiments auf dem Verum-Kompartiment versehen ist.

7. TTS nach einem der Ansprüche 4 bis 6, **dadurch gekennzeichnet, dass** die Steuermembran ausgewählt ist aus der Gruppe der Polymerfolien aus Polyethylenen, Polypropylenen, Silikonen, Polyurethanen und der Copolymere aus Polyethylen und Polyvinylacetat.

8. TTS nach Anspruch 6 oder 7, **dadurch gekennzeichnet, dass** die Haftkleberschicht unter der Steuermembran ausgewählt ist aus der Gruppe der Silikone und der Copolymeren aus Polyethylen und Polyvinylacetat, klebend gemacht unter Zusatz von bestimmten Klebharzen.

9. TTS nach einem der Ansprüche 6 bis 8, **dadurch gekennzeichnet, dass** die Haftkleberschicht eine Steuerwirkung ausübt, wobei die Steuerwirkung von ihrer Schichtdicke abhängt.

10. TTS nach einem der Ansprüche 6 bis 9, **dadurch gekennzeichnet, dass** die Haftkleberschicht auf Basis Copolymeren aus Polyethylen und Polyvinylacetat mit Klebharzzusätzen ihre Steuerwirkung über das Verhältnis von Polyethylen zu Polyvinylacetat ausübt.

11. TTS nach einem der Ansprüche 1 bis 10, **dadurch gekennzeichnet, dass** das Flüssig-Reservoirsystem zusätzlich zumindest ein Absorptionsmittel enthält, das aus der Gruppe ausgewählt ist, die aus Cyclodextrinen, Polyvinylpyrrolidonen und Cellulosederivaten besteht.

12. TTS nach einem der Ansprüche 1 bis 11, **dadurch gekennzeichnet, dass** das Flüssig-Reservoirsystem zusätzliche Hilfsstoffe im Sinne von Verdickungsmitteln wie Mineralöle, Wollwachse, Polyacrylsäure, hochmolekulare Polyethylenglykole oder feindisperses Siliciumdioxid enthält.

13. TTS nach einem der Ansprüche 1 bis 12, **dadurch gekennzeichnet, dass** das Verum-Kompartiment eine haftklebende Polymermatrix umfasst, die aus haftklebenden Polymeren auf Basis von Acrylsäure und/oder Methacrylsäure sowie deren Estern, Polyacrylaten, Isobutylen, Ethylen-Vinylacetat, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymere oder Heißschmelzklebern bestehen, oder auf der Basis von haftklebenden Silikonpolymeren oder Polysiloxanen, besonders bevorzugt auf Basis von aminresistenten Polydimethylsiloxanen hergestellt wurde.

14. TTS nach einem der Ansprüche 1 bis 13, **dadurch gekennzeichnet, dass** es sich bei der inneren Rückschicht um eine Polyesterfolie handelt, die mit Löchern versehen ist.

15. Verum-Kompartiment zur Verwendung in Kombination mit einem Enhancer-Kompartiment in einem TTS nach einem der vorhergehenden Ansprüche, umfassend eine wirkstoffhaltige, haftklebende Polymermatrix, die mit einer wirkstoffundurchlässigen, ablösbaren Schutzschicht bedeckt ist, und eine innere Rückschicht, bei der es sich um eine poröse oder nicht poröse Schicht mit hoher Permeabilität für den/die zu verwendenden Permeationsenhancer handelt, **dadurch gekennzeichnet, dass** der Wirkstoff in vollständig kristallisierter Form in der Polymermatrix vorliegt, und dass es eine äußere Rückschicht und eine ablösbare Schutzschicht aufweist, die wirkstoffundurchlässig sind und das Verum-Kompartiment abdecken.

16. Verum-Kompartiment nach Anspruch 15, **dadurch gekennzeichnet, dass** die haftklebende Polymermatrix aus haftklebenden Polymeren auf Basis von Acrylsäure und/oder Methacrylsäure sowie deren Estern, Polyacrylaten, Isobutylen, Ethylen-Vinylacetat, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymere oder Heißschmelzklebern bestehen, oder auf der Basis von haftklebenden Silikonpolymeren oder Polysiloxanen, besonders bevorzugt auf Basis von aminresistenten Polydimethylsiloxanen hergestellt wurden.

17. Verum-Kompartiment nach Anspruch 15 oder 16, **dadurch gekennzeichnet, dass** die innere Rückschicht perforiert ist.

18. Verum-Kompartiment nach einem der Ansprüche 15 bis 17, **dadurch gekennzeichnet, dass** es sich bei der inneren Rückschicht um eine Polyesterfolie handelt, die mit Löchern versehen ist, oder um eine Folie auf Basis von Polyethylenterephthalat.

19. Verum-Kompartiment nach Anspruch 18, **dadurch gekennzeichnet, dass** das Material für die äußere Rückschicht und ablösbare Schutzschicht aus der Gruppe ausgewählt ist, die aus Polyester, Polyvinylchlorid, Ethylenvinylacetat, Vinylacetat, Polyethylen, Polypropylen, Cellulosederivaten ausgewählt ist

20. Verum-Kompartiment nach Anspruch 15 oder 19, **dadurch gekennzeichnet, dass** die äußere Rückschicht und/oder ablösbare Schutzschicht durch geeignete Oberflächenbehandlung, beispielsweise Silikonisierung, ablösbar gemacht wurde.

21. Verum-Kompartiment nach einem der Ansprüche 15 oder 19 bis 23, **dadurch gekennzeichnet, dass** die äußere Rückschicht mit einer zusätzlichen Auflage versehen ist.

22. Verum-Kompartiment nach Anspruch 21, **dadurch gekennzeichnet, dass** es sich bei der zusätzlichen Auflage um eine Bedampfung mit Metallen oder anderen diffusionssperrenden Zusatzstoffen wie Siliciumdioxid oder Aluminiumoxid handelt.

23. Verum-Kompartiment nach einem der Ansprüche 15 bis 17, **dadurch gekennzeichnet, dass** die innere Rückschicht auf Basis von Polyethylenterephthalat mit einer Haftfolie auf Basis von Polyurethan, Polyisobutylen oder Polyacrylaten als äußere Rückschicht abgedeckt ist.

24. Verum-Kompartiment nach einem der Ansprüche 15 oder 19 bis 23, **dadurch gekennzeichnet, dass** es sich bei der ablösbaren Schutzschicht um Polytetrafluorethylen-behandeltes Papier, Cellophan oder Polyvinylchlorid handelt.

25. Enhancer-Kompartiment zur Verwendung in Kombination mit einem Verum-Kompartiment in einem TTS nach einem der Ansprüche 1 bis 14, **dadurch gekennzeichnet, dass** ein beutelförmiges Flüssig-Reservoirsystem, in dem ein oder mehrere Permeationsenhancer in flüssiger Form, als Gel, Paste oder Lösung enthalten sind, und eine für Wirkstoff und Permeationsenhancer undurchlässige Rückschicht umfaßt, wobei das Flüssig-Reservoirsystem eine Steuermembran aufweist, mit der die Freisetzung des/der Permeationsenhancer kontrolliert wird, und das Flüssig-Reservoirsystem zusätzlich zumindest ein Absorptionsmittel enthält, das ausgewählt ist aus der Gruppe bestehend ausCyclodextrinen, Polyvinylpyrrolidonen und Cellulosederivaten.

26. Enhancer-Kompartiment nach Anspruch 25, **dadurch gekennzeichnet, dass** der/die Permeationsenhancer aus der Gruppe der niederen, einwertigen, höheren einwertigen und mehrwertigen Alkohole), monosubstituierten Ester von mehrwertigen Alkoholen, Terpene, Terpenalkohole, Ester mittlerer Carbonsäuren, Polyoxyethylenfettalkohole, Polyoxyethylenfettalkoholether, Polyoxyethylen-fettsäureester, Partialfettsäureester der Sorbitane, Dimethylisosorbid, Dimethylsulfoxid, Ölsäure sowie pharmazeutisch verträglichen flüssigen Stickstoffverbindungen wie N-Methylpyrrolidon, Diethyltoluamid, Dimethylen-propylenharnstoff, Diethanolamin ausgewählt ist/sind.

27. Enhancer-Kompartiment nach Anspruch 25 oder 26, **dadurch gekennzeichnet, dass** die Hülle des Flüssig-Reservoirsystems, zumindest auf der dem Verum-Kompartiment zugewandten Seite, aus der Steuermembran besteht, oder zusätzlich auf der dem Verum-Kompartiment zugewandten Seite des Flüssig-Reservoirsystems aufgebracht ist.

28. Enhancer-Kompartiment nach einem der Ansprüche 25 bis 27, **dadurch gekennzeichnet, dass** die Steuermembran selbstklebend oder mit einer Haftkleberschicht zur Befestigung des Enhancer-Kompartiments auf dem Verum-Kompartiment versehen ist.

29. Enhancer-Kompartiment nach einem der Ansprüche 25 bis 28, **dadurch gekennzeichnet, dass** die Steuermembran aus der Gruppe der Polymerfolien aus Polyethylenen, Polypropylenen, Silikonen, Polyurethanen und der Copolymere aus Polyethylen und Polyvinylacetat ausgewählt ist.

30. Enhancer-Kompartiment nach einem der Ansprüche 25 bis 29, **dadurch gekennzeichnet, dass** die Haftkleberschicht unter der Steuermembran ausgewählt ist aus der Gruppe der Silikone und der Copolymeren aus Polyethylen und Polyvinylacetat, klebend gemacht unter Zusatz von bestimmten Klebharzen.

31. Enhancer-Kompartiment nach Anspruch 28, **dadurch gekennzeichnet, dass** die Haftkleberschicht auf Basis Copolymeren aus Polyethylen und Polyvinylacetat mit Klebharzzusätzen ihre Steuerwirkung über das Verhältnis Polyethylen zu Polyvinylacetat ausübt.

32. Enhancer-Kompartiment nach einem der Ansprüche 25 bis 31, **dadurch gekennzeichnet, dass** das Flüssig-Reservoirsystem zusätzliche Hilfsstoffe im Sinne von Verdickungsmitteln wie Mineralöle, Wollwachse, Polyacrylsäure, hochmolekulare Polyethylenglykole oder feindisperses Siliziumdioxid enthält.

33. Enhancer-Kompartiment nach einem der Ansprüche 25 bis 32, **dadurch gekennzeichnet, dass** es zusätzlich eine ablösbare Schutzfolie aufweist.

34. Verfahren zur Herstellung eines TTS nach einem der Ansprüche 1 bis 14, **dadurch gekennzeichnet, dass** ein Enhancer-Kompartiment gemäß einem der Ansprüche 25 bis 33 als Überpflaster auf ein Verum-Kompartiment gemäß einem der Ansprüche 15 bis 24 geklebt wird.

## Claims

1. Transdermal therapeutic system (TTS) comprising a first compartment and a second compartment, where
- the first compartment comprises a pressure-sensitive adhesive polymer matrix which is provided on its side facing away from the skin with an inner backing layer and in which an active ingredient in a solid state of aggregation is present, and one side of which serves to attach the system to the skin and is covered with an active ingredient-impermeable, detachable protective layer which is located on the side opposite the inner backing layer;
- the second compartment comprises a liquid reservoir system which comprises one or more permeation enhancers in liquid form, as gel, paste or solution, where the specified active ingredient is at least partly soluble in the permeation enhancer or at least in one of the permeation enhancers;
and where the two compartments are stored separately from one another prior to use and are only joined together for application to the human skin in such a way that the side of the first, active ingredient-containing compartment which is opposite the side covered with the detachable protective layer is connected with the second, enhancer-containing compartment, and the diffusion of the enhancer(s) into the active ingredient-containing compartment is permitted in a controlled manner via a control membrane such that a continuously oversaturated system with a maximum thermodynamic activity is formed;
and where verum compartment and enhancer compartment, after they have been joined together, are separated from one another by an "inner backing layer", which is a layer with high permeability for the permeation enhancer(s).

2. TTS according to Claim 1, **characterized in that** the permeation enhancer(s) from the enhancer compartment is/are selected from the group comprising lower monohydric, higher monohydric and polyhydric alcohols, monosubstituted esters of polyhydric alcohols, terpenes, terpene alcohols, esters of medium carboxylic acids, polyoxyethylene fatty alcohols, polyoxyethylene fatty alcohol ethers, polyoxyethylene fatty acid esters, partial fatty acid esters of the sorbitans, dimethyl isosorbide, dimethyl sulphoxide, oleic acid, and pharmaceutically compatible liquid nitrogen compounds such as N-methylpyrrolidone, diethyltoluamide, dimethylenepropyleneurea, diethanolamine.

3. TTS according to Claim 2, **characterized in that** the permeation enhancers from the enhancer compartment are present singly or as mixtures with one another.

4. TTS according to one of Claims 1 to 3, **characterized in that** the permeation enhancers from the enhancer compartment are released into the verum compartment in a controlled manner via a control membrane.

5. TTS according to Claim 4, **characterized in that** the covering of the liquid reservoir system, at least on the side facing the verum compartment, consists of the control membrane, or is additionally applied to the side of the liquid reservoir system facing the verum compartment.

6. TTS according to Claim 4 or 5, **characterized in that** the control membrane is self-adhesive or is provided with a pressure-sensitive adhesive layer for attaching the enhancer compartment to the verum compartment.

7. TTS according to one of Claims 4 to 6, **characterized in that** the control membrane is selected from the group of the polymer films of polyethylenes, polypropylenes, silicones, polyurethanes and the copolymers of polyethylene and polyvinyl acetate.

8. TTS according to Claim 6 or 7, **characterized in that** the pressure-sensitive adhesive layer underneath the control membrane is selected from the group of silicones and the copolymers of polyethylene and polyvinyl acetate, rendered adhesive with the addition of certain adhesive resins.

9. TTS according to one of Claims 6 to 8, **characterized in that** the pressure-sensitive adhesive layer exerts a control effect, the control effect depending on its layer thickness.

10. TTS according to one of Claims 6 to 9, **characterized in that** the pressure-sensitive adhesive layer based on copolymers of polyethylene and polyvinyl acetate with adhesive resin additives exerts its control effect via the ratio of polyethylene to polyvinyl acetate.

11. TTS according to one of Claims 1 to 10, **characterized in that** the liquid reservoir system additionally comprises at least one absorption agent which is selected from the group consisting of cyclodextrins, polyvinylpyrrolidones and cellulose derivatives.

12. TTS according to one of Claims 1 to 11, **characterized in that** the liquid reservoir system comprises additional auxiliaries in the sense of thickeners such as mineral oils, wool waxes, polyacrylic acid, high molecular weight polyethylene glycols or finely dispersed silicon dioxide.

13. TTS according to one of Claims 1 to 12, **characterized in that** the verum compartment comprises a pressure-sensitive adhesive polymer matrix which consists of pressure-sensitive adhesive polymers based on acrylic acid and/or methacrylic acid, and esters thereof, polyacrylates, isobutylene, ethylene-vinyl acetate, natural and/or synthetic rubbers, for example acrylonitrile-butadiene rubber, butyl rubber or neoprene rubber, styrene-diene copolymers such as styrene-butadiene block copolymers or hot-melt adhesives, or has been produced on the basis of pressure-sensitive adhesive silicone polymers or polysiloxanes, particularly preferably on the basis of amine-resistant polydimethylsiloxanes.

14. TTS according to one of Claims 1 to 13, **characterized in that** the inner backing layer is a polyester film provided with perforations.

15. Verum compartment for use in combination with an enhancer compartment in a TTS according to one of the preceding claims, comprising an active ingredient-containing, pressure-sensitive adhesive polymer matrix which is covered with an active ingredient-impermeable, detachable protective layer and an inner backing layer, which is a porous or un-porous layer with high permeability for the permeation enhancer(s) to be used, **characterized in that** the active ingredient is present in the polymer matrix in completely crystallized form, and that it has an outer backing layer and a detachable protective layer which are active ingredient-impermeable and cover the verum compartment.

16. Verum compartment according to Claim 15, **characterized in that** the pressure-sensitive adhesive polymer matrix consists of pressure-sensitive adhesive polymers based on acrylic acid and/or methacrylic acid, and esters thereof, polyacrylates, isobutylene, ethylene-vinyl acetate, natural and/or synthetic rubbers, for example acrylonitrile-butadiene rubber, butyl rubber or neoprene rubber, styrene-diene copolymers such as styrene-butadiene block copolymers or hot-melt adhesives, or has been produced on the basis of pressure-sensitive adhesive silicone polymers or polysiloxanes, particularly preferably on the basis of amine-resistant polydimethylsiloxanes.

17. Verum compartment according to Claim 15 or 16, **characterized in that** the inner backing layer is perforated.

18. Verum compartment according to one of Claims 15 to 17, **characterized in that** the inner backing layer is a polyester film provided with perforations or a film based on polyethylene terephthalate.

19. Verum compartment according to Claim 18, **characterized in that** the material for the outer backing layer and detachable protective layer is selected from the group consisting of polyester, polyvinyl chloride, ethylene vinyl acetate, vinyl acetate, polyethylene, polypropylene and cellulose derivatives.

20. Verum compartment according to Claim 15 or 19, **characterized in that** the outer backing layer and/or detachable protective layer has been rendered detachable by suitable surface treatment, for example siliconization.

21. Verum compartment according to one of Claims 15 or 19 to 23, **characterized in that** the outer backing layer is provided with an additional overlay.

22. Verum compartment according to Claim 21, **characterized in that** the additional overlay is a vapour deposition with metals or other diffusion-blocking additives such as silicon dioxide or aluminium oxide.

23. Verum compartment according to one of Claims 15 to 17, **characterized in that** the inner backing layer based on polyethylene terephthalate is covered with an adhesive film based on polyurethane, polyisobutylene or polyacrylates as outer backing layer.

24. Verum compartment according to one of Claims 15 or 19 to 23, **characterized in that** the detachable protective layer is polytetrafluoroethylene-treated paper, cellophane or polyvinyl chloride.

25. Enhancer compartment for use in combination with a verum compartment in a TTS according to one of Claims 1 to 14, **characterized in that** it comprises a bag-shaped liquid reservoir system in which one or more permeation enhancers are present in liquid form, as gel, paste or solution, and a backing layer that is impermeable to active ingredient and permeation enhancers, where the liquid reservoir system has a control membrane with which the release of the permeation enhancer(s) is controlled, and the liquid reservoir system additionally comprises at least one absorption agent which is selected from the group consisting of cyclodextrins, polyvinylpyrrolidones and cellulose derivatives.

26. Enhancer compartment according to Claim 25, **characterized in that** the permeation enhancer(s) is/are selected from the group of lower monohydric, higher monohydric and polyhydric alcohols, monosubstituted esters of polyhydric alcohols, terpenes, terpene alcohols, esters of medium carboxylic acids, polyoxyethylene fatty alcohols, polyoxyethylene fatty alcohol ethers, polyoxyethylene fatty acid esters, partial fatty acid esters of the sorbitans, dimethyl isosorbide, dimethyl sulphoxide, oleic acid, and pharmaceutically compatible liquid nitrogen compounds such as N-methylpyrrolidone, diethyltoluamide, dimethylenepropyleneurea, diethanolamine.

27. Enhancer compartment according to Claim 25 or 26, **characterized in that** the covering of the liquid reservoir system consists, at least on the side facing the verum compartment, of the control membrane, or is additionally applied to the side of the liquid reservoir system facing the verum compartment.

28. Enhancer compartment according to one of Claims 25 to 27, **characterized in that** the control membrane is self-adhesive or is provided with a pressure-sensitive adhesive layer for attaching the enhancer compartment to the verum compartment.

29. Enhancer compartment according to one of Claims 25 to 28, **characterized in that** the control membrane is selected from the group of the polymer films made of polyethylenes, polypropylenes, silicones, polyurethanes and the copolymers of polyethylene and polyvinyl acetate.

30. Enhancer compartment according to one of Claims 25 to 29, **characterized in that** the pressure-sensitive adhesive layer beneath the control membrane is selected from the group of the silicones and the copolymers made of polyethylene and polyvinyl acetate, rendered adhesive with the addition of certain adhesive resins.

31. Enhancer compartment according to Claim 28, **characterized in that** the pressure-sensitive adhesive layer based on copolymers of polyethylene and polyvinyl acetate with adhesive resin additives exerts its control effect via the ratio of polyethylene to polyvinyl acetate.

32. Enhancer compartment according to one of Claims 25 to 31, **characterized in that** the liquid reservoir system comprises additional auxiliaries in the sense of thickeners such as mineral oils, wool waxes, polyacrylic acid, high molecular weight polyethylene glycols or finely dispersed silicon dioxide.

33. Enhancer compartment according to one of Claims 25 to 32, **characterized in that** it additionally has a detachable protective film.

34. Method for producing a TTS according to one of Claims 1 to 14, **characterized in that** an enhancer compartment according to one of Claims 25 to 33 is adhered as over-plaster to a verum compartment according to one of Claims 15 to 24.

## Revendications

1. Système thérapeutique transdermique (TTS), comprenant un premier compartiment et un second compartiment, dans lequel :
- le premier compartiment comprend une matrice polymère autoadhésive, qui est pourvue, sur son côté opposé à la peau, d'une couche de verso interne et qui contient un principe actif présent à l'état d'agrégat solide, et dont une face sert à la fixation du système sur la peau et est revêtue d'une couche de protection enlevable perméable au principe actif, qui se trouve sur la face opposée à la couche de verso interne ;
- le second compartiment comprend un système de réservoir de liquide, qui contient un ou plusieurs promoteurs de perméation sous la forme d'un liquide, d'un gel, d'une pâte ou d'une solution, dans lequel le principe actif mentionné est au moins partiellement soluble dans le promoteur de perméation ou au moins dans l'un des promoteurs de perméation ; et
dans lequel les deux compartiments sont conservés séparément l'un de l'autre avant leur utilisation et ne sont assemblés l'un à l'autre que pour l'application sur la peau humaine de sorte que la face du premier compartiment contenant le principe actif, qui est opposée à la face revêtue de la couche de protection enlevable, soit liée au second compartiment contenant le ou les promoteurs et que la diffusion du ou des promoteurs soit permise de manière contrôlée dans le compartiment contenant le principe actif via une membrane de commande, afin de former un système sursaturé en continu avec une activité thermodynamique maximale ; et
dans lequel le compartiment de fait et le compartiment promoteur sont séparés l'un de l'autre après leur assemblage par une « couche de verso interne » qui est une couche de perméabilité élevée pour le ou les promoteurs de perméation.

2. TTS selon la revendication 1, **caractérisé en ce que** le ou les promoteurs de perméation du compartiment promoteur est ou sont choisis dans le groupe qui comprend les alcools monovalents inférieurs ainsi que les alcools monovalents et polyvalents supérieurs, les esters monosubstitués d'alcools polyvalents, les terpènes, les alcools terpéniques, les esters d'acides carboxyliques moyens, les alcools gras de polyoxyéthylène, les éthers d'alcools gras de polyoxyéthylène, les esters d'acides gras de polyoxyéthylène, les esters d'acides gras partiels des sorbitans, l'isosorbide de diméthyle, le sulfoxyde de diméthyle, l'acide oléique ainsi que les composés d'azote liquides pharmaceutiquement compatibles, tels que la N-méthylpyrrolidone, le diéthyltoluamide, le diméthylène-propylène-urée et la diéthalonamine.

3. TTS selon la revendication 2, **caractérisé en ce que** les promoteurs de perméation du compartiment promoteur sont présents individuellement ou sous la forme de mélanges.

4. TTS selon l'une quelconque des revendications 1 à 3, **caractérisé en ce que** les promoteurs de perméation du compartiment promoteur sont délivrés via une membrane de commande de manière contrôlée dans le compartiment de fait.

5. TTS selon la revendication 4, **caractérisé en ce que** l'enveloppe du système de réservoir de liquide est constituée, au moins sur la face tournée vers le compartiment de fait, de la membrane de commande ou est appliquée en plus sur la face du système de réservoir de liquide tournée vers le compartiment de fait.

6. TTS selon la revendication 4 ou 5, **caractérisé en ce que** la membrane de commande est autocollante ou est pourvue d'une couche autocollante pour fixer le compartiment promoteur sur le compartiment de fait.

7. TTS selon l'une quelconque des revendications 4 à 6, **caractérisé en ce que** la membrane de commande est choisie dans le groupe des films polymères constitués des polyéthylènes, des polypropylènes, des silicones, des polyuréthanes et des copolymères de polyéthylène et de poly(acétate de vinyle).

8. TTS selon la revendication 6 ou 7, **caractérisé en ce que** la couche autocollante qui se trouve en dessous de la membrane de commande est choisie dans le groupe des silicones et des copolymères de polyéthylène et de poly(acétate de vinyle) adhésivés par addition de certaines résines adhésives.

9. TTS selon l'une quelconque des revendications 6 à 8, **caractérisé en ce que** la couche autocollante exerce un effet de commande, l'effet de commande dépendant de son épaisseur de couche.

10. TTS selon l'une quelconque des revendications 6 à 9, **caractérisé en ce que** la couche autocollante à base de copolymères de polyéthylène et de poly(acétate de vinyle) avec des additions de résine adhésive exerce son effet de commande sur le rapport du polyéthylène au poly(acétate de vinyle).

11. TTS selon l'une quelconque des revendications 1 à 10, **caractérisé en ce que** le système de réservoir de liquide contient en outre au moins un agent absorbant, qui est choisi dans le groupe constitué des cyclodextrines, des polyvinylpyrrolidones et des dérivés cellulosiques.

12. TTS selon l'une quelconque des revendications 1 à 11, **caractérisé en ce que** le système de réservoir de liquide contient des substances auxiliaires supplémentaires au sens d'agents épaississants tels que les huiles minérales, le suint, l'acide polyacrylique, les polyéthylèneglycols de poids moléculaires élevés ou le dioxyde de silicium finement dispersé.

13. TTS selon l'une quelconque des revendications 1 à 12, **caractérisé en ce que** le compartiment de fait comprend une matrice polymère autocollante, qui est constituée de polymères autocollants à base d'acide acrylique et/ou d'acide méthacrylique ainsi que de leurs esters, de polyacrylates, d'isobutylène, d'éthylène-acétate de vinyle, de caoutchoucs naturels et/ou synthétiques, par exemple de caoutchouc d'acrylonitrile-butadiène, de caoutchouc de butyle ou de caoutchouc de néoprène, de copolymères de styrène-diène, tels que les copolymères séquencés de styrène-butadiène ou les colles thermofusibles, ou sont fabriqués sur la base de polymères de silicone ou de polysiloxanes autocollants, tout particulièrement à base de polydiméthylsiloxanes résistant aux amines.

14. TTS selon l'une quelconque des revendications 1 à 13, **caractérisé en ce que** la couche de verso interne est un film de polyester qui est pourvu de trous.

15. Compartiment de fait pour utilisation en combinaison avec un compartiment promoteur dans un TTS selon l'une quelconque des revendications précédentes, comprenant une matrice polymère autocollante contenant un principe actif, qui est revêtue d'une couche de protection enlevable imperméable au principe actif, et une couche de verso interne qui est une couche poreuse ou non poreuse avec une perméabilité élevée pour le ou les promoteurs de perméation à utiliser, **caractérisé en ce que** le principe actif est présent sous une forme complètement cristallisée dans la matrice polymère, et **en ce qu'**il présente une couche de verso externe et une couche de protection enlevable, qui sont imperméables au principe actif et qui recouvrent le compartiment de fait.

16. Compartiment de fait selon la revendication 15, **caractérisé en ce que** la matrice polymère autocollante est constituée de polymères autocollants à base d'acide acrylique et/ou d'acide méthacrylique ainsi que de leurs esters, de polyacrylates, d'isobutylène, d'éthylène-acétate de vinyle, de caoutchoucs naturels et/ou synthétiques, par exemple de caoutchouc d'acrylonitrile-butadiène, de caoutchouc de butyle ou de caoutchouc de néoprène, de copolymères de styrène-diène, tels que les copolymères séquencés de styrène-butadiènes ou les colles thermofusibles, ou sont fabriqués à base de polymères de silicone ou de polysiloxanes autocollants, tout particulièrement à base de polydiméthylsiloxanes résistant aux amines.

17. Compartiment de fait selon la revendication 15 ou 16, **caractérisé en ce que** la couche de verso interne est perforée.

18. Compartiment de fait selon l'une quelconque des revendications 15 à 17, **caractérisé en ce que** la couche de verso interne est un film de polyester qui est pourvu de trous, ou un film à base de poly(téréphtalate d'éthylène).

19. Compartiment de fait selon la revendication 18, **caractérisé en ce que** le matériau pour la couche de verso externe et la couche de protection enlevable est choisi dans le groupe constitué du polyester, du poly(chlorure de vinyle), de l'éthylène-acétate de vinyle, de l'acétate de vinyle, du polyéthylène, du polypropylène et de dérivés de celluloses.

20. Compartiment de fait selon la revendication 15 ou 19, **caractérisé en ce que** la couche de verso externe et/ou la couche de protection enlevable a ou ont été rendues enlevables par un traitement superficiel approprié, par exemple, par siliconage.

21. Compartiment de fait selon l'une quelconque des revendications 15 ou 19 à 23, **caractérisé en ce que** la couche de verso externe est pourvue d'un revêtement supplémentaire.

22. Compartiment de fait selon la revendication 21, **caractérisé en ce que** le revêtement supplémentaire est une vaporisation avec des métaux ou d'autres additifs barrières à la diffusion, tels que le dioxyde de silicium ou l'oxyde d'aluminium.

23. Compartiment de fait selon l'une quelconque des revendications 15 à 17, **caractérisé en ce que** la couche de verso interne à base de poly(téréphtalate d'éthylène) est revêtue, comme couche de verso externe, d'un film collant à base de polyuréthane, de polyisobutylène ou de polyacrylates.

24. Compartiment de fait selon l'une quelconque des revendications 15 ou 19 à 23, **caractérisé en ce que** la couche de protection enlevable est du papier traité au polytétrafluoroéthylène, de la cellophane ou du poly(chlorure de vinyle).

25. Compartiment promoteur pour utilisation en combinaison avec un compartiment de fait dans un TTS selon l'une quelconque des revendications 1 à 14, **caractérisé en ce qu'**un système de réservoir de liquide en forme de sachet, dans lequel est ou sont contenus un ou plusieurs promoteurs de perméation sous forme de liquide, de gel, de pâte ou de solution, comprend une couche de verso imperméable au principe actif et au promoteur de perméation, dans lequel le système de réservoir de liquide présente une membrane de commande avec laquelle la libération du ou des promoteurs de perméation est contrôlée et le système de réservoir de liquide contient en plus au moins un agent absorbant qui est choisi dans le groupe constitué des cyclodextrines, des polyvinylpyrrolidones et des dérivés cellolosiques.

26. Compartiment promoteur selon la revendication 25, **caractérisé en ce que** le ou les promoteurs de perméation est ou sont choisis dans le groupe qui comprend les alcools monovalents inférieurs ainsi que les alcools monovalents et polyvalents supérieurs, les esters monosubstitués d'alcools polyvalents, les terpènes, les alcools terpéniques, les esters d'acides carboxyliques moyens, les alcools gras de polyoxyéthylène, les éthers d'alcools gras de polyoxyéthylène, les esters d'acides gras de polyoxyéthylène, les esters d'acides gras partiels des sorbitans, l'isosorbide de diméthyle, le sulfoxyde de diméthyle, l'acide oléique ainsi que les composés d'azote liquides pharmaceutiquement compatibles, tels que la N-méthylpyrrolidone, le diéthyltoluamide, le diméthylène-propylène-urée et la diéthalonamine.

27. Compartiment promoteur selon la revendication 25 ou 26, **caractérisé en ce que** l'enveloppe du système de réservoir de liquide est constituée, au moins sur la face tournée vers le compartiment de fait, de la membrane de commande ou est appliquée en plus sur la face du système de réservoir de liquide tournée vers le compartiment de fait.

28. Compartiment promoteur selon l'une quelconque des revendications 25 à 27, **caractérisé en ce que** la membrane de commande est autocollante ou est pourvue d'une couche autocollante pour fixer le compartiment promoteur au compartiment de fait.

29. Compartiment promoteur selon l'une quelconque des revendications 25 à 28, **caractérisé en ce que** la membrane de commande est choisie dans le groupe des films polymères constitués de polyéthylènes, de polypropylènes, de silicones, de polyuréthanes et des copolymères de polyéthylène et de poly(acétate de vinyle).

30. Compartiment promoteur selon l'une quelconque des revendications 25 à 29, **caractérisé en ce que** la couche autocollante en dessous de la membrane de commande est choisie dans le groupe des silicones et des copolymères de polyéthylène et de poly(acétate de vinyle) adhésivés par addition de certaines résines adhésives.

31. Compartiment promoteur selon la revendication 28, **caractérisé en ce que** la couche autocollante à base de copolymères de polyéthylène et de poly(acétate de vinyle) avec des additions de résine adhésive, exerce son effet de commande sur le rapport du polyéthylène au poly(acétate de vinyle).

32. Compartiment promoteur selon l'une quelconque des revendications 25 à 31, **caractérisé en ce que** le système de réservoir de liquide contient des substances auxiliaires supplémentaires au sens d'agents épaississants tels que les huiles minérales, le suint, l'acide polyacrylique, les polyéthylèneglycols de poids moléculaires élevés ou le dioxyde de silicium finement dispersé.

33. Compartiment promoteur selon l'une quelconque des revendications 25 à 32, **caractérisé en ce qu'**il présente en plus un film de protection enlevable.

34. Procédé de fabrication d'un TTS selon l'une quelconque des revendications 1 à 14, **caractérisé en ce qu'**un compartiment promoteur selon l'une des revendications 25 à 33 est collé sous forme de pansement de recouvrement sur un compartiment de fait selon l'une quelconque des revendications 15 à 24.
